# FASCICULE DE BREVET EUROPEEN

(11) **EP 0 498 685 B1**
(45) Date de publication et mention de la délivrance du brevet: **07.12.1994**
(21) Numéro de dépôt: 92400066.4
(22) Date de dépôt: 10.01.1992
(51) Int. Cl.: A61F 2/34

(54) **Cupule cotyloidienne expansible**
Ausdehnbare Hüftgelenkspfanne
Expansible acetabular cup

(30) Priorité: 06.02.1991 FR 9101320
(43) Date de publication de la demande: 12.08.1992
(73) Titulaire: SCIENCE ET MEDECINE (Société anonyme), F-75014 Paris (FR); Sejourne, Pierre, F-13300 Salon-de-Provence (FR)
(72) Inventeur: Sejourne, Pierre, F-13300 Salon-de-Provence (FR); Breard, Francis Henri, F-75014 Paris (FR)

(56) Documents cités:
- EP-A- 0 242 633
- EP-A- 0 353 171
- DE-A- 3 726 213
- FR-A- 2 645 433

## Description

La présente invention concerne une cupule cotyloïdienne, de forme générale hémisphérique, présentant, découpées dans sa paroi, des languettes radialement déployables qui se raccordent au sommet de la cupule, sensiblement le long d'un parallèle de cette dernière, et portent, sur leur surface extérieure, des aspérités pour l'ancrage de la cupule dans la paroi d'un cotyle.

De telles cupules couramment dénommées cupules expansibles ou à expansion, constituent la garniture extérieure d'endoprothèses cotyloïdiennes, qu'elles permettent d'implanter dans la paroi du cotyle sans l'utilisation d'un ciment chirurgical. On élimine ainsi les risques de déscellement ultérieur.

Les divers types de cupules expansibles (voir par exemple EP-A-242633), connues à ce jour, sont cependant loin de donner satisfaction. En premier lieu, on constate qu'au cours de l'ancrage, les endoprothèses, revêtues de ces cupules, ne parviennent pas à rester au contact du fond de la cavité du cotyle et font ainsi l'objet, après ancrage total, d'un défaut d'emboîtement relativement prononcé. En outre, lors de sa mise en place initiale, la cupule, ayant ses languettes rétractées, ne porte que par son sommet contre le fond du cotyle et, de ce fait, ne peut être orientée et centrée correctement dans la cavité de ce dernier, ce qui peut occasionner ultérieurement des luxations.

En définitive, les endoprothèses actuelles, munies de cupules expansibles, ne permettent pas une reconstitution anatomique convenable du cotyle, ce qui se traduit la plupart du temps par des désordres fonctionnels ultérieurs dans la prothèse articulaire totale, en particulier dans le cas d'une prothèse de hanche.

La présente invention se propose de remédier à ces inconvénients et, pour ce faire, elle a pour objet une cupule cotyloïdienne, du type spécifié en introduction, qui se caractérise en ce que les aspérités de ses languettes consistent en des dents d'ancrage dont le flanc intérieur, tourné vers le sommet de la cupule, est formé en contre-dépouille sur la surface de la languette qui les porte.

Grâce à cette contre-dépouille qui équivaut à une légère inclinaison de leur flanc intérieur par rapport à la direction radiale, les dents d'ancrage, lors de leur pénétration dans l'os sous l'effet du déploiement des languettes, auront tendance à maintenir l'endoprothèse plaquée contre le fond du cotyle, de sorte que le défaut d'emboîtement constaté jusqu'à présent pourra être réduit dans une mesure appréciable, voire totalement supprimé.

Pour garantir cependant un emboîtement plaçant l'endoprothèse cotyloïdienne sensiblement dans sa position anatomique, il est avantageusement prévu que les dents d'ancrage, réparties sur plusieurs parallèles de la cupule, présentent, sur un même parallèle, un angle de contre-dépouille sensiblement identique, et que cet angle qui, sur chaque dent, est défini, dans un plan de coupe de celle-ci passant par un méridien de la cupule, entre la droite délimitant le flanc intérieur de la dent et la tangente à la surface de la languette, au point d'intersection de cette droite avec ladite surface, décroisse progressivement d'un parallèle au suivant en direction du bord libre de la languette. En pratique, l'angle, ainsi défini, de contre-dépouille des dents d'ancrage peut varier entre 80° et 60° environ, sur des cupules standard.

La position anatomique optimale sera alors obtenue avec un mode de réalisation préféré de la cupule selon l'invention dans lequel l'angle définissant la contre-dépouille de chaque dent d'ancrage est, dans son plan de coupe méridien, obtenu par tangence du flanc intérieur de la dent avec un cercle imaginaire ayant son centre situé sensiblement sur le parallèle le long duquel la languette se raccorde au sommet de la cupule. Dans ce cas en effet, lorsque les dents d'ancrage pénètrent dans la paroi du cotyle, leur flanc intérieur épouse une trajectoire parfaitement concentrique avec celle suivie par la languette en cours de déploiement, et n'occasionnent donc aucun déport vers le bas de l'endoprothèse, la maintenant ainsi en contact intime avec le fond du cotyle.

Selon une autre caractéristique de l'invention, la paroi de la cupule comprend encore des branches qui, à partir du sommet de la cupule, s'étendent chacune entre deux languettes respectives et sont solidaires au-delà de ces dernières, d'éléments de paroi définissant la base de la cupule, de préférence constituée par un anneau circulaire continu auquel toutes les branches sont réunies.

Les languettes sont ainsi retenues à l'intérieur d'une cage rigide qui permet, sans difficulté, d'orienter et de centrer correctement la cupule dans le cotyle, lors du positionnement préliminaire de cette dernière précédant l'ancrage, pour ainsi éliminer les risques ultérieurs de luxation.

La présente invention va maintenant être décrite plus en détail, sur la base d'un mode de réalisation préféré, nais non-limitatif, illustré par les dessins annexés sur lesquels :
- la figure 1 est une vue en plan d'une cupule conforme à ce mode de réalisation de l'invention ;
- la figure 2 en est une vue de côté, pour moitié en coupe transversale selon la ligne II-II de la figure 1 ;
- la figure 3 est une vue en coupe partielle de la cupule, illustrant la caractéristique principale de l'invention ; et
- la figure 4 représente en coupe une endoprothèse cotyloïdienne, munie de la cupule des figures 1 et 2, en place dans le cotyle du bassin.

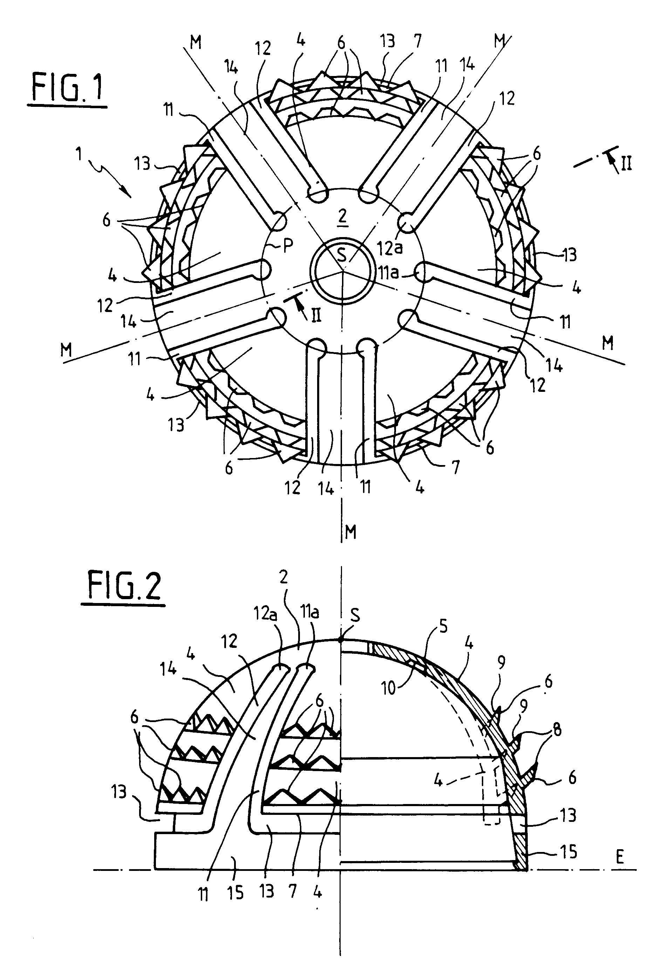

La cupule 1, exemplifiée sur les figures 1 et 2, est traditionnellement réalisée en un alliage de titane à mémoire de forme, sous une configuration d'ensemble hémisphérique.

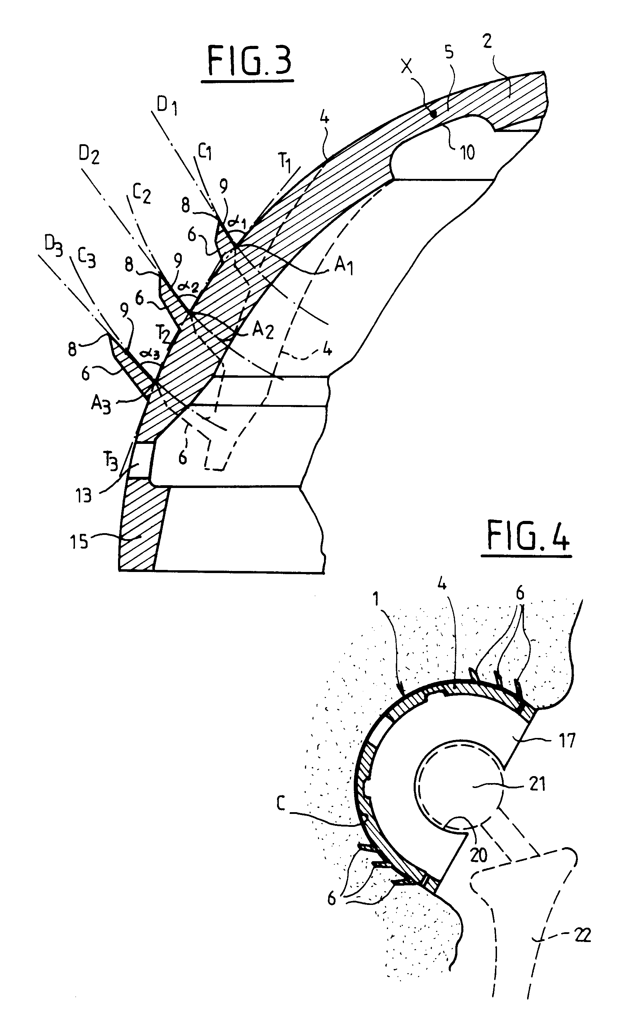

A partir d'un parallèle P délimitant le sommet 2, en forme de calotte sphérique, de la cupule 1, non loin de son pôle S, la paroi relativement mince de cette dernière est découpée en plusieurs languettes radiales 4. Ces languettes 4, ici au nombre de cinq, identiques en forme et dimensions, peuvent être légèrement déployées depuis leur court segment de raccordement 5 au sommet 2 de la cupule, qui, comme le montrent les figures 2 et 3, est défini à cheval sur le parallèle P, par une rainure 10 usinée dans la face intérieure de la cupule. La surface extérieure des languettes 4 est par ailleurs garnie d'aspérités 6 pour l'ancrage de la cupule 1 dans la paroi osseuse du cotyle destiné à la recevoir.

Dans l'exemple représenté, les aspérités 6 des languettes 4 sont réalisées sous la forme de dents triangulaires et, comme le montre la figure 1, chaque languette 4 est pourvue de quatre rangées de trois dents 6, formées, à intervalles égaux, sur des parallèles de la cupule et regroupées sur la moitié de la languette proche de son bord libre 7. Chaque dent 6 est en outre positionnée symétriquement par rapport à un plan méridien de la cupule, tel que le plan de coupe de la figure 3, ici commun à trois dents.

La figure 3, sur laquelle on peut voir que les dents 6 sont effilées et biseautées en pointe à leur extrémité 8, montre en outre que leur flanc intérieur 9, tourné vers le sommet de la cupule 1, est formé en contre-dépouille, c'est-à-dire légèrement incliné vers la surface de la languette 4. Cette contre-dépouille, vue dans le plan méridien de symétrie de chaque dent d'ancrage 6, est plus précisément définie par l'angle α₁, α₂ ou α₃ formé entre la droite D₁, D₂ ou D₃ définissant le flanc intérieur 9 de la dent et la tangente T₁, T₂ ou T₃ à la surface de la languette 4, au point d'intersection A₁, A₂ ou A₃ de la droite D₁, D₂ ou D₃ avec cette dernière.

Il est à noter que cet angle α₁, α₂, α₃ décroît légèrement avec la longueur de l'arc de méridien s'étendant entre le point A₁, A₂, A₃, précédemment défini, et le parallèle P de la cupule, autour duquel la languette 4 est susceptible de se déployer et, de façon optimale, cette décroissance est obtenue par tangence, audit point A₁, A₂, A₃, du flanc intérieur 9 de chaque dent 6 avec un cercle imaginaire C₁, C₂ ou C₃ tracé dans le plan méridien de symétrie de la dent 6, depuis un centre X situé sur le parallèle P à mi-épaisseur du serpent de raccordement 5 de la languette 4.

On précisera ici que les angles α₁, α₂, α₃ ainsi définis ont des valeurs d'environ 73° pour le premier, 69° pour le deuxième et 65° pour le troisième.

En revenant à la figure 1, on observera par ailleurs que les languettes 4 sont définies latéralement par une paire respective de fentes 11,12 pratiquées chacune dans un plan sensiblement parallèle à un plan méridien M de la cupule 1 et légèrement décalé par rapport à celui-ci, ces fentes se prolongeant jusqu'à proximité du plan équatorial E de la cupule où elles sont réunies par une troisième fente 13 ménagée sensiblement le long d'un parallèle de cette dernière.

De la sorte, les languettes 4 alternent, sur le pourtour de la cupule 1, avec des branches d'enveloppe sphérique, telles que 14, venues de matière, d'un côté, avec la calotte 2 de cette dernière et, de l'autre côté, avec un anneau circulaire commun 15 définissant, au-delà des bords libres 7 des languettes, la base de la cupule.

On observera aussi que les fonds 11a,12a des fentes méridiennes 11,12, sont conformés en découpes circulaires élargies, à chaque extrémité des serpents de raccordement 5 des languettes 4.

La cupule 1, qui vient d'être décrite, constitue l'élément extérieur d'une endoprothèse cotyloïdienne de la hanche, dont elle enveloppe le corps intérieur 17 réalisé en un matériau auto-lubrifiant et muni d'une cavité sphérique 20 de réception de la tête 21 d'une prothèse fémorale 22, comme représenté sur la figure 4, laquelle montre l'endoprothèse en place dans le cotyle naturel C du bassin, préalablement usiné aux dimensions extérieures de la cupule.

Lors de la première phase de mise en place de la cupule 1, réalisée de façon classique, les branches 14 de cette dernière, avec la calotte 2 et l'anneau 15, forment, pour les languettes 4, une cage rigide qui permet, sans difficulté, d'orienter et de centrer correctement, dans le cotyle C, la cupule 1 dont les languettes 4 auront préalablement été légèrement rétractées à l'intérieur de cette cage, comme représenté en traits interrompus sur les figures 2 et 3.

Ensuite, sous l'effet du court déploiement des languettes 4 autour du parallèle P, obtenu par introduction du corps intérieur 17 de l'endoprothèse dans la cupule, les dents d'ancrage 6 viennent mordre dans la paroi osseuse du cotyle C et leur flanc intérieur 9 y pénètre en suivant une trajectoire en arc de cercle C₁, C₂, C₃, centrée sur le parallèle P autour duquel se déploient les languettes. De la sorte, la pénétration des dents 6 dans la paroi du cotyle n'engendre aucun déport vers le bas de l'endoprothèse et celle-ci reste donc plaquée contre le fond du cotyle et parfaitement bien emboîtée dans la cavité de ce dernier.

## Revendications

1. Cupule cotyloïdienne (1), de forme générale hémisphérique, présentant, découpées dans sa paroi, des languettes radialement déployables (4) qui se raccordent au sommet (2) de la cupule, sensiblement le long d'un parallèle de cette dernière, et portent, sur leur surface extérieure, des aspérités (6) pour l'ancrage de la cupule dans la paroi du cotyle, caractérisée en ce que ces aspérités consistent en des dents d'ancrage (6) dont le flanc intérieur (9), tourné vers le sommet (2) de la cupule, est formé en contre-dépouille sur la surface de la languette (4) qui les porte.

2. Cupule selon la revendication 1, caractérisée en ce que les dents d'ancrage (6), réparties sur plusieurs parallèles de la cupule (1), présentent, sur un même parallèle, un angle de contre-dépouille sensiblement identique, et en ce que cet angle (α₁, α₂, α₃) qui, sur chaque dent, est défini, dans un plan de coupe de celle-ci passant par un méridien de la cupule, entre la droite (D₁,D₂,D₃) délimitant le flanc intérieur (9) de la dent et la tangente (T₁,T₂,T₃) à la surface de la languette (4), au point d'intersection (A₁,A₂,A₃) de cette droite avec ladite surface, décroit progressivement d'un parallèle au suivant en direction du bord libre (7) de la languette (4).

3. Cupule selon la revendication 2, caractérisée en ce que l'angle de contre-dépouille (α₁, α₂, α₃) des dents d'ancrage (6) varie entre 80° et 60° environ.

4. Cupule selon la revendication 2 ou 3, caractérisée en ce que l'angle (α₁, α₂, α₃) définissant la contre-dépouille de chaque dent d'ancrage (6) est, dans son plan méridien de coupe, obtenu par tangence du flanc intérieur (9) de la dent avec un cercle imaginaire (C₁,C₂,C₃) ayant son centre situé sensiblement sur le parallèle (P) le long duquel la languette (4) se raccorde au sommet (2) de la cupule.

5. Cupule selon l'une quelconque des revendications 1 à 4, caractérisée en ce que sa paroi (3) comprend des branches (14) qui, à partir du sommet (2) de la cupule, s'étendent chacune entre deux languettes respectives (4) et sont solidaires au-delà de ces dernières, d'éléments de paroi (15) constituant la base de la cupule.

6. Cupule selon la revendication 5, caractérisée en ce que ladite base est constituée par un anneau circulaire continu (15) auquel toutes les branches (14) sont réunies.

## Claims

1. Acetabular cup (1), with a general hemispherical form, presenting, cut in its wall, radially spreadable tongues (4) which are connected to the top (2) of the cup, practically all along a parallel of the latter, and having, on their external surface, asperities (6) to anchor the cup in the wall of the acetabulum, characterized by the fact that these asperities consist of anchoring teeth (6) whose internal side (9), turned towards the top (2) of the cup, is formed in a re-entrant angle on the surface of the tongue (4) which carries them.

2. Cup according to claim 1, characterized by the fact that the anchoring teeth (6), distributed over several parallels of the cup (1), present, on the same parallel, an almost identical re-entrant angle, and by the fact that this angle (α1, α2, α3) which, on each tooth, is defined, in a cutting plane of the latter, passing through a meridian of the cup, between the straight line (D1, D2, D3) delimiting the internal side (9) of the tooth and the tangent (T1, T2, T3) to the surface of the tongue (4), up to the point of intersection (A1, A2, A3) of this straight line with the aforesaid surface, decreases progressively from one parallel to the next in the direction of the free edge (7) of the tongue (4).

3. Cup according to claim 2, characterized by the fact that the re-entrant angle (α1, α2, α3) of the anchoring teeth (6) varies between about 80° and 60°.

4. Cup according to claim 2 or 3, characterized by the fact that the angle (α1, α2, α3) defining the undercut of each anchoring tooth (6) is, in its meridian cutting plane, obtained by tangency of the internal side (9) of the tooth with an imaginary circle (C1, C2, C3) whose center is situated practically on the parallel (P) along which the tongue (4) is connected to the top (2) of the cup.

5. Cup according to any one of claims 1 to 4, characterized by the fact that its wall (3) comprises branches (14) which, from the top (2) of the cup, each extend between two respective tongues (4) and are integral after the latter, with the elements of the wall (15) constituting the base of the cup.

6. Cup according to claim 5, characterized by the fact that the aforesaid base is made up of a continuous circular band (15) to which all of the branches (14) are joined.

## Patentansprüche

1. Allgemein halbkugelförmige Gelenkpfannenkuppel (1), die radial auseinanderfaltbare, aus ihrer Wand ausgeschnittene Zungen (4) aufweist, die an der Kuppelspitze (2) verbunden sind, im wesentlichen entlang einer Parallele zu der Kuppel, und an ihrer Außenseite Unebenheiten (6) aufweisen, die zur Verankerung der Kuppel in der Pfannenwand dienen, **dadurch gekennzeichnet,** daß diese Unebenheiten aus Verankerungszähnen (6) bestehen, deren Innenfläche (9), die zur Kuppelspitze (2) zeigt, als Hinterschneidung an der Oberfläche der die Zähne tragenden Zungen (4) ausgebildet ist.

2. Kuppel nach Anspruch 1, **dadurch gekennzeichnet,** daß die Verankerungszähne (6), die auf mehrere Parallelen der Kuppel (1) aufgeteilt sind, an derselben Parallele einen praktisch identischen Hinterschneidungswinkel aufweisen und daß dieser Winkel (α 1, α2, α3), der in der entsprechenden Schnittebene jedes Zahnes, die über den Meridian der Kuppel führt, zwischen der Geraden (D1, D2, D3), die die Innenseite (9) des Zahns begrenzt und der Tangente (T1, T2,T3) an der Oberfläche der Zunge (4) am Schnittpunkt (A1, A2, A3) dieser Geraden mit der genannten Fläche festgelegt ist, von einer Parallelen zur anderen nach und nach und in Richtung des freien Randes (7) der Zunge (4) nach und nach kleiner wird.

3. Kuppel nach Anspruch 2, **dadurch gekennzeichnet**, daß der Hinterschneidungswinkel (α 1, α2, α3) der Verankerungszähne (6) etwa zwischen 80° und 60° variiert.

4. Kuppel nach Anspruch 2 oder 3, **dadurch gekennzeichnet**, daß der Winkel (α 1, α2, α3), der die Hinterschneidung jedes Verankerungszahns (6) festlegt, in seiner meridionalen Schnittebene durch Berührung der Innenseite (9) des Zahnes mit einem gedachten Kreis (C1, C2, C3), dessen Mittelpunkt sich ziemlich genau auf der Parallelen (P) befindet, an deren Längsseite die Zunge (4) mit der Kuppelspitze (2) verbunden ist, erhalten wird.

5. Kuppel nach einem der Ansprüche 1 bis 4, **dadurch gekennzeichnet**, daß die Kuppelwand (3) Zweige (14) aufweist, die, ausgehend von der Kuppelspitze (2), jeweils zwischen zwei Zungen (4) entlanglaufen und über die Zungen hinaus miteinander verbunden sind, wobei die Wandelemente (15) die Unterfläche der Kuppel darstellen.

6. Kuppel nach Anspruch 5, **dadurch gekennzeichnet,** daß die genannte Unterfläche aus einem durchgehenden Ring (15) besteht, an den alle Zweige (14) angeschlossen sind.
